(19) Europäisches Patentamt
European Patent Office
Office européen des brevets

(11) Publication number: **0 155 221 A2**

# (12) EUROPEAN PATENT APPLICATION

(21) Application number: 85630016.5

(22) Date of filing: 26.02.85

(51) Int. Cl.[4]: C 07 C 93/14, C 07 C 149/267, C 07 D 277/72

(30) Priority: 27.02.84 US 583921

(43) Date of publication of application: 18.09.85 Bulletin 85/38

(84) Designated Contracting States: DE FR GB IT NL

(71) Applicant: THE GOODYEAR TIRE & RUBBER COMPANY, 1144 East Market Street, Akron, Ohio 44316 (US)

(72) Inventor: D'Sidocky, Richard Michael, 3050 Clearview Road, Ravenna Ohio 44266 (US)

(74) Representative: Weyland, Joseph Jean Pierre, Goodyear International Tire Technical Center Patent Department Avenue Gordon Smith, L-7750 Colmar-Berg (LU)

## (54) A process for the preparation of functionalized monomers.

(57) There is disclosed a process for the modification of a monomer comprising contacting said monomer with an alkali ionizable molecule containing chemical functionality in the presence of a quaternary ammonium or phosphonium salt and thereafter contacting the resultant mixture with an alkaline compound. The resulting functionalized monomer can then be polymerized without purification to yield a polymer that contains a covalently bonded functionality.

EP 0 155 221 A2

# A PROCESS FOR THE PREPARATION OF FUNCTIONALIZED MONOMERS

## Technical Field:

This invention is concerned with the preparation of functionalized monomers and their polymerization to yield polymers that have covalently bonded chemical functionality.

## Background of the Invention

It is well known to add antidegradant compounds to polymers in order to stabilize the polymer against oxidation by air or certain oxidizing substances. One of the problems of antidegradant technology has been the physical loss of antidegradants from the polymer or elastomer, chiefly through volatilization and/or extraction. It has been necessary to utilize antidegradants that apart from having good antidegradant activity also possess good solubility in the polymer. The problems of volatilization and extraction have been somewhat overcome by the development of higher molecular weight antidegradants. Several known antidegradants are available which have a molecular weight near 1,000.

There are two known methods by which chemical or covalent bonding of a functionality, more specifically an antidegradant to a polymer, can be accomplished. One is the direct reaction of an antidegradant, or an incipient antidegradant, with an elastomer and the other is incorporation of an antidegradant during formation of an elastomer. An example of the first method is the reaction of aromatic nitroso compounds with natural rubber. Another general method which has been used to chemically bond an antidegradant onto an elastomer is incorporation of an antidegradant monomer during formation of the elastomer. This has been done by using polymerizable antidegradants; that is compounds which contain both an antidegradant function and a polymerizable function, e.g. a vinyl group.

Heretofore, the polymerizable antidegradant has been tediously prepared, purified and then added as a monomer to the polymerization recipe. Other functionalities such as synergists, accelerators, plasticizers, fungicides, etc., have been attached to polymers so as to prevent their migration or volatilization. There exists the need for a process which will provide for the efficient and economic preparation of functionalized monomers.

## Disclosure of the Invention

The present invention provides an improved process for the production of monomers containing covalently bonded functionalities. The inventors have unexpectedly found that a compound containing a vinyl moiety and a halo-benzyl moiety can be modified by contacting the compound with a quaternary ammonium or phosphonium salt and an alkali ionizable molecule containing the desired chemical functionality; thereafter, contacting the resultant mixture with an alkaline compound. Through the instant process one can produce a monomer having a covalently bonded chemical functionality. The functionalized monomer can then be polymerized without purification in a conventional emulsion polymerization to yield a polymer containing covalently bonded chemical functionality.

There is also disclosed a process for the modification of a monomer having a vinyl moiety and a halo-benzyl moiety comprising; (A) contacting said monomer with a quaternary ammonium or phosphonium salt and an alkaline ionizable molecule containing anti-degradant functionality, thereafter, (B) contacting the resultant mixture with an alkaline compound at a temperature of 10°-120°C in the presence of an inert atmosphere.

There is further disclosed a process for the production of a functionalized monomer, wherein said monomer contains a polymerizable vinyl moiety and a halo-benzyl moiety, said process comprising:

(A) contacting said monomer with a quaternary salt selected from the group of salts having the structural formula:

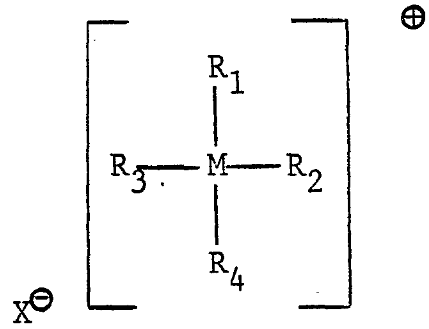

wherein M is nitrogen or phosphorous; $X^{\ominus}$ is selected from the group of radicals consisting of chloride, bromide, fluoride, iodide, acetate, alkoxide and hydroxide; $R_1$, $R_2$, $R_3$ and $R_4$ are mono- valent hydrocarbon radicals where the total number of carbon atoms in $R_1$, $R_2$, $R_3$ and $R_4$ is 12 to 70; and

(B) an alkali ionizable molecule selected from the group consisting of 4-mercaptopropionamide diphenylamine, 2,6-di-t-butyl-4-mercaptophenol, 4-mercaptophenol, p-hydroxydiphenylamine, N-(4-anilinophenyl)-2-mercaptoacetamide, 2,6-di-t-butylhydroquinone, dodecyl mercaptopropionate; thereafter, (C) contacting the resultant mixture with an alkaline compound selected from the group consisting of NaOH, $NaHCO_3$, KOH, $K_2CO_3$, $Na_2CO_3$, $KHCO_3$, NaH, KH, LiH, Na, Li, K, NaOH, $K_2CO_3$, $Na_2CO_3$ and $K_2HCO_3$;

at a temperature of 10°-120°C in the presence of an inert atmosphere.

The present invention is a process which is conducted by stirring two liquid phases; an organic phase containing the monomer to be functionalized and a concentrated aqueous phase of the anion to be transferred, all in the presence of a quaternary ammonium or phosphonium salt. The modified or functionalized monomer is then homo-polymerized or co-polymerized without purification to yield a polymer having the desired covalently bonded pendant functionality.

The modified monomer prepared according to the present invention is polymerized with other monomers or mixtures of monomers capable of homopolymerization, copolymerization or interpolymerization by free-radical mechanism. Suitable monomers include conjugated dienes containing four to ten carbon atoms. Examples of such monomers are 1,3-butadiene; 2-ethyl-1,3-butadiene; 2,3-dimethyl-1,3-butadiene; isoprene, piperylene; 1,3-hexadienes; 1,3-decadienes; and vinyl monomers including styrene, α-methyl styrene, divinylbenzene, and acrylonitrile.

It should be understood that only one functionality is loaded on the monomer, however, different kinds of functionalized monomers may be reacted together to give polymers having multiple pendant functionalities.

Representative of the quaternary salts that are useful in the present invention are compounds of the general structural formula:

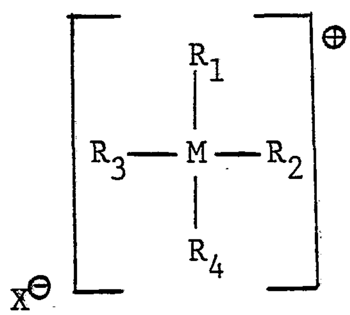

(I)

wherein M is nitrogen or phosphorus; $X^{\ominus}$ is a radical selected from the group of chloride, bromide, fluoride, iodide, acetate, alkoxide and hydroxide and $R_1$, $R_2$, $R_3$ and $R_4$ are monovalent hydrocarbon radicals where the total of carbon atoms in $R_1$, $R_2$, $R_3$ and $R_4$ is 12 to 70 carbon atoms. Some examples of compound (I) are tetrabutyl ammonium bromide, trioctyl methyl ammonium chloride, tri-n-butyl hexadecyl phosphonium bromide, tri-n-butyl methyl ammonium chloride, tetraheptyl ammonium iodide and tricaprylyl methyl ammonium chloride.

Additional information on quaternary salts can be found in <u>Phase Transfer Catalysis Principles and Techniques</u>

by C. Stark and C. Liotta, Chapter 3, Page 57, Academic Press, 1978, and Phase Transfer Catalysis in Organic Synthesis by W. Weber and G. Gokel, Chapter 1, Springer-Verlag, 1977, both incorporated herein by reference.

The monomer to be functionalized is reacted in the presence of an alkaline solution. Examples of aqueous alkaline solutions useful in the present invention are aqueous solutions of NaOH, $NaHCO_3$, KOH, $K_2CO_3$, $Na_2CO_3$ and $KHCO_3$.

Strong nonaqueous solutions can be used as an alternative to aqueous alkaline solutions. Examples of the alkaline compounds in the nonaqueous state are NaH, KH, LiH, Na, Li, K, NaOH, $NaHCO_3$, KOH, $K_2CO_3$, $Na_2CO_3$ and $K_2HCO_3$.

The temperature at which the reaction is carried out is 10°C but should not exceed 120°C with a temperature of from 60° to 80°C being preferred.

It is advantageous to exclude oxygen from the reaction with the halomethylated monomer, however, small amounts of oxygen, for example oxygen present as an impurity in commercial grade nitrogen can be tolerated. Thus, the skilled artisan would be able to utilize the instant invention in conjunction with an appropriate inert atmosphere.

The functionality with which the monomer is to be modified is presented in the form of an alkali ionizable molecule. Numerous chemical functionalities such as accelerators, synergists, dyes, flame retardants and fungicides can be used if in the form of an alkali ionizable molecule.

The alkali ionizable molecules containing antidegradant properties are of particular interest. These include those antidegradants of the amine and the hindered phenolic classes. These antidegradants are generally known by the term chain-breaking antioxidants, however, other antioxidants commonly known as peroxide-decomposing antioxidants, ultraviolet screening agents, triplet quenchers and metal

deactivators are contemplated herein. Some examples of chain-breaking antioxidants are represented by the following formulas:

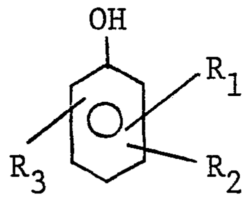

II

where $R_1$ and $R_2$ are the same or different radicals selected from the group consisting of hydrogen, alkyl, cycloalkyl, aralkyl or aryl and wherein $R_3$ is selected from

-SH

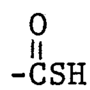

,

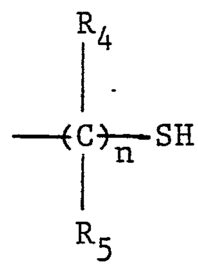

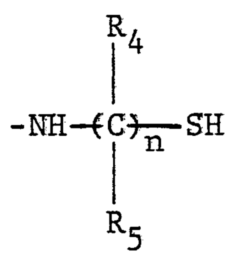

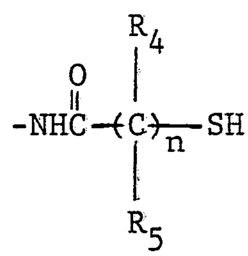

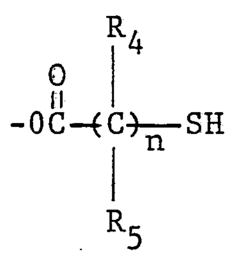

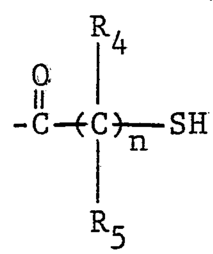

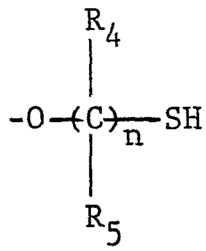

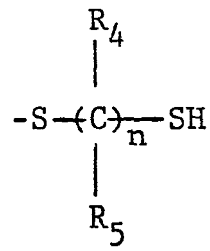

where $R_4$ and $R_5$ are both hydrogen, n equals 1-12; or when $R_4$ is hydrogen $R_5$ is a hydrocarbon radical having from 1 to 20 carbon atoms and n equals 1 or $R_4$ and $R_5$ are the same or different radical selected from the group consisting of hydrocarbon radicals having 1 to 10 carbon atoms and n equals 1.

Other alkali ionizable molecules having antidegradant properties useful in this invention are:

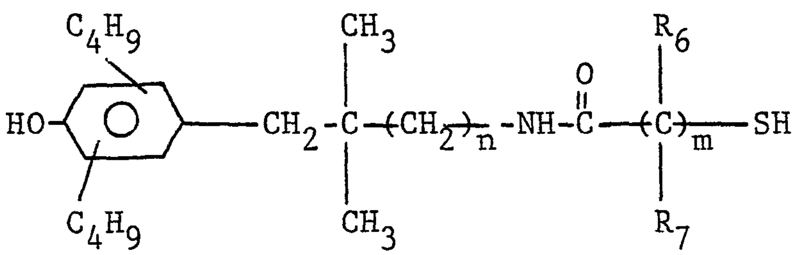

III

wherein n equals 0 or 1, $R_6$ and $R_7$ are the same or different radicals selected from the group consisting of hydrogen or hydrocarbon radicals having 1 to 20 carbon atoms and m is 1 or 2; and

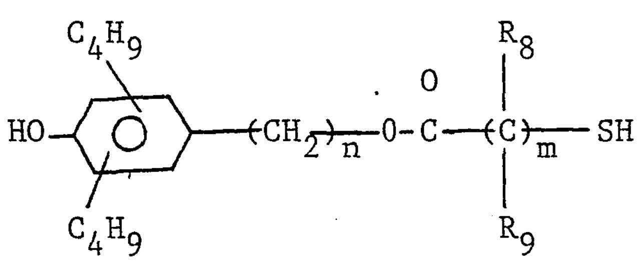

IV

wherein m is equal to 1 or 2, n is 1-12, $R_8$ and $R_9$ are the same or different and selected from hydrogen or hydrocarbon radicals having from 1 to 20 carbon atoms; and

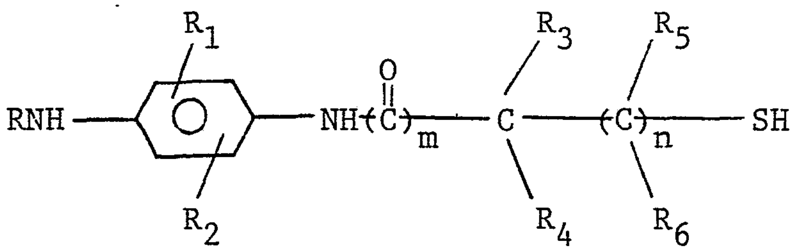 V

wherein m equals 0 or 1; $R_3$ and $R_4$ are the same radicals selected from the group consisting of hydrogen or alkyl radicals having 1 to 5 carbon atoms and n equals 0 or 1, $R_5$ and $R_6$ are the same radicals selected from the group consisting of hydrogen or alkyl radicals having 1 to 5 carbon atoms and n equals 1; wherein $R_1$ and $R_2$ are the same or different radicals selected from the group consisting of hydrogen and alkyl radicals having 1 to 5 carbon atoms; and R is a radical selected from the group consisting of a cycloalkyl radical having from 5 to 12 carbon atoms, a branched acyclic group comprising a chain of 1 to 12 carbon atoms and each carbon may be substituted with 1 to 2 alkyl groups of 1 to 3 carbon atoms, an alicyclic aralkyl or aryl radical having from 7 to 14 carbon atoms, and a phenyl radical or phenyl radical which is substituted in any one or more positions with an alkyl or alkoxy group of 1 to 4 carbon atoms or with a radical of the formula:

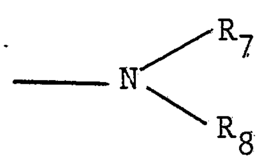

in which both $R_7$ and $R_8$ may be either a hydrogen radical or an alkyl radical of 1 to 4 carbon atoms; and

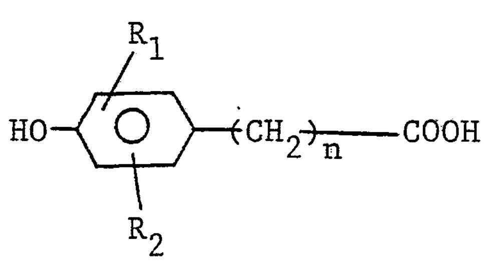 VI

wherein n is equal to 0 to 12 and wherein $R_1$ and $R_2$ are the same or different radicals selected from the group comprising hydrogen, and hydrocarbon radicals having 1 to 18 carbon atoms; and

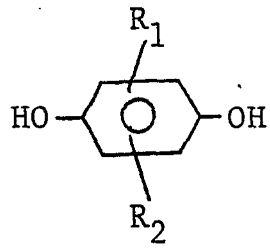

VII

wherein $R_1$ and $R_2$ are the same or different radicals selected from the group comprising hydrogen and hydrocarbon radicals having 1 to 18 carbon atoms, and

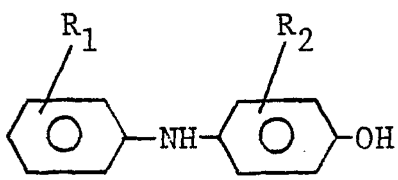

VIII

wherein $R_1$ and $R_2$ are the same or different radicals selected from the group consisting of hydrogen and hydrocarbon radicals having 1 to 18 carbon atoms.

Through the process of the present invention these alkali ionizable molecules are reacted with the halo-methylated monomer to yield a monomer for polymerization which has chemically attached to it these antidegradant functionalities. Other functions can be attached to the halo-methylated monomer to yield functionalized monomers such as:

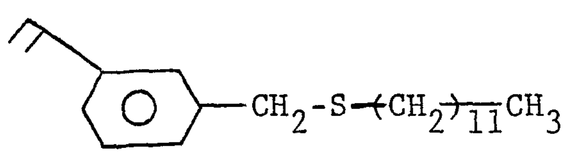

for use as a monomer to yield a polymer containing a chemically bound plasticizer, or

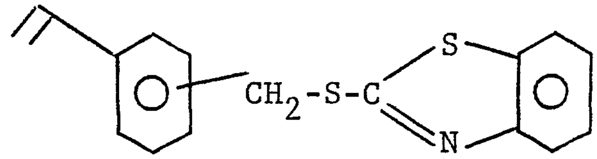

as a polymer-bound accelerator, or

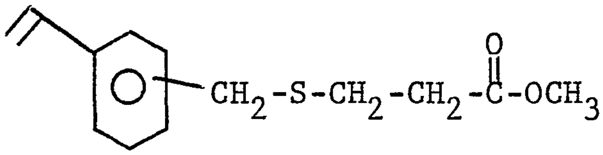

as a synergist, or

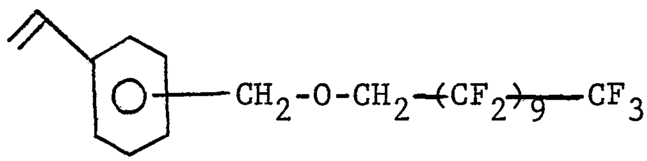

as a lubricant, or

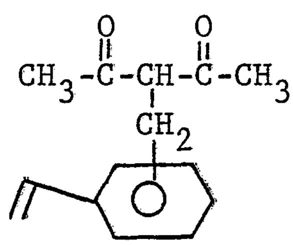

as a metal deactivator. Other functionalities such as antioxidant stabilizers, flame retardants, bacteriocides, fungicides, and dyes can be chemically bound to a polymer through the process of the instant invention wherein the starting monomer is effectively and efficiently modified prior to polymerization with other monomers.

The following examples are supplied in order to illustrate, but not necessarily to limit, the process of the present invention.

## Examples 1-4

Four 115 ml (4 ounce) bottles were each charged with 15 parts (3 grams) styrene, 6.15 parts (1.23 grams, 6.64 millimoles) 4-hydroxydiphenylamine, 5.05 parts (1.01 grams, 6.64 millimoles) vinylbenzyl chloride, 0.27 grams (6.64 millimoles) NaOH, .21 grams $Bu_4NBr$ (10 mole percent based on hydroxydiphenylamine) and 150 parts water (30 grams). The bottles were tumbled overnight (≈18 hours) at 60°C in a rotary bath resulting in greater than 90% vinylbenzyl chloride conversion to the 4-anilinophenylvinylbenzyl ether monomer. To the bottles containing the preformed monomer, 100 parts of 10% aqueous sodium lauryl sulphonate surfactant, two parts of catalyst (2,2' azobisisobutyronitrile), 0.6 parts of modifier (tertiary dodecyl mercaptan) and 75 parts of butadiene were charged. The polymerizations were conducted for varying periods of time at 60°C, see Table I. In Example 3, sixty parts of dichloromethane based on total monomers was added. Conversions of 82-96 percent of monomers were obtained in 24 hours for Examples 1-3. When these bottles were allowed to remain in the bath over a total of 89 hours, 98-100 percent conversions were obtained.

The amount in parts of bound antioxidant was determined after the samples were placed in a thimble in a Soxhlet extractor and extracted with methanol for 48 hours. The samples were removed, dried under an aspirator at room temperature and were analyzed.

## TABLE I

| Parts of Example | Monomer Ratio (parts) Bd/Styrene/Preformed Monomer/$CH_2Cl_2$ | | | | Conversion % (Hrs) 16 | 17.5 | 24 | 39.5 | 89 | Bound Antioxidant |
|---|---|---|---|---|---|---|---|---|---|---|
| 1 | 75 | 15 | 10 | 0 | | 72.7 | 81.0 | | 98.5 | 8.5 |
| 2 | 75 | 15 | 10 | 0 | | 69.0 | 82.5 | | 100 | 10 |
| 3 | 75 | 15 | 10 | 60 | | 88.7 | 96.2 | | 100 | 10 |
| 4 | 75 | 15 | 10 | 0 | 51.8 | | | 90.1 | | 10 |

To determine the effectiveness of monomers prepared according to the invention oxygen absorption tests were conducted on SBR-1006 polymers with polymer bound hydroxydiphenylamine and unbound hydroxydiphenylamines. The testing procedure is described in detail in Industrial and Engineering Chemistry, Vol. 43, p. 456 (1951) and Vol. 45, p. 392 (1953). A sufficient amount of hydroxydiphenylamine was dissolved in toluene and added to an appropriate amount of SBR-1006 dissolved in toluene to yield a polymer after evaporation of the toluene containing 1.25 parts per hundred of hydroxydiphenylamine per 100 parts rubber. The same procedure was used for the 4-anilinophenylvinylbenzyl ether monomer yielding a physical admixture of the monomer and rubber. The polymer from Example 4 in latex form was mixed with SBR latex to yield after coagulation and extraction a polymer containing 1.25 parts bound 4-anilinophenylvinylbenzyl ether. Table II sets out the data.

TABLE II

Oxygen Absorption, SBR, 100°C

| Antioxidant | Parts A.O. | Hours to Absorb 1% $O_2$ by weight |
|---|---|---|
| Hydroxydiphenylamine (physical admixture w/SBR 1006) | 1.25 | 185 |
| 4-anilinophenylvinylbenzyl ether monomer (physical admixture w/SBR 1006) | 1.25 | 526 |
| Polymer Bound Hydroxydiphenylamine (extracted) | 1.25 | 680 |

This data demonstrates that even after extraction the bound antioxidant provides excellent antioxidative protection.

## Example 5

### Preparation of dodecyl vinylbenzylthiopropionate

To a reaction flask was added 80 gms dodecyl mercaptopropionate, 5 mole% tetrabutylammonium bromide, 44 ml of a 30% aqueous sodium hydroxide solution and 200 ml toluene. After stirring for one hour, 50 gms vinylbenzyl chloride was added and the reaction mixture heated at 60°C overnight under nitrogen. Analysis of the reaction mixture showed 94% vinylbenzyl chloride functionalization. The organic layer was washed twice with water (65°C) and then diluted with an equal volume of hexane followed by recrystallization.

## Example 6

### Preparation of vinylbenzyl dodecyl sulfide

To a reaction flask was charged 30.4 gms vinylbenzyl chloride, 44.5 gms 1-dodecanethiol, 5 mole% tetrabutylammonium bromide, 50 gms of a 40% aqueous sodium hydroxide solution and 120 ml toluene. The reaction was run overnight at 60°C under nitrogen. Analysis showed complete conversion of vinylbenzyl chloride to vinylbenzyl dodecyl sulfide. The reaction mixture was diluted with water and the organic layer washed with 25% aqueous sodium hydroxide to remove the excess 1-dodecanethiol. After several washes with saturated aqueous sodium chloride, the organic layer was dried over anhydrous sodium sulfate, stripped, and the product recrystallized from hexane.

## Example 7

### Preparation of vinylbenzyl dodecyl sulfide

A 115 ml (4 oz) bottle was charged with 6 gms styrene, 1.92 gms vinylbenzyl chloride, 2.54 gms dodecanethiol, 0.5 gm sodium hydroxide, 30 ml of water and 5 mole% tetrabutylammonium bromide. The bottle was purged with nitrogen and tumbled for 20.5 hours at 60°C in a polymerization bath. Analysis showed 81.7% conversion of the vinylbenzyl chloride to vinylbenzyl dodecyl sulfide.

The same reaction run without tetrabutylammonium bromide yielded only 14.3% vinylbenzyl dodecyl sulfide after 20.5 hours reaction.

## Example 8

### Preparation of (Vinylbenzyl)-2-mercaptobenzothiazole

To a two liter flask was charged 40 g vinylbenzyl chloride, 47.8 gm 2-mercaptobenzothiazole, 100 gms of a 40% aqueous sodium hydroxide solution, 5 mole% tetrabutylammonium bromide and 500 ml of toluene. The contents were placed under nitrogen and reacted at 60°C overnight. Analysis showed complete conversion of vinylbenzyl chloride to (vinylbenzyl)-2-mercaptobenzothiazole. The organic layer was washed twice with 5% sodium hydroxide solution followed by water washes. The organic layer was dried over anhydrous sodium sulfate, stripped and the product recrystallized from hexane.

## Example 9

### Preparation of (vinylbenzyl)-2-mercaptobenzothiazole

A 115 ml (4 oz) bottle was charged with 8gms styrene, 1.18 gms 2-mercaptobenzothiazole, 1.08 gms vinylbenzyl chloride, 0.282 gm sodium hydroxide, 30 ml of water and 5 mole% tetrabutylammonium bromide. The bottle was purged with nitrogen and tumbled for two hours at 60°C in a polymerization bath. Analysis showed quantitative conversion to the desired (vinylbenzyl)-2-mercaptobenzothiazole.

The same reaction run without tetrabutylammonium bromide yielded only 52% (vinylbenzyl)-2-mercaptobenzothiazole after two hours reaction.

## Example 10

### Use of Dodecyl vinylbenzylthiopropionate as a polymer bound synergist

Dodecyl vinylbenzylthiopropionate (DVBTP) prepared as in Example 5 was polymerized with styrene and butadiene to yield a modified SBR latex that was 36.5% solids and contained 5 parts per hundred by weight polymer bound dodecyl vinylbenzylthiopropionate. The DVBTP modified SBR latex was blended with unmodified SBR latex to give a final latex blend containing 0.5 phr bound DVBTP. The latex mixture was coagulated using isopropyl alcohol. The isolated crumbs were dissolved in chloroform followed by re-precipitation in isopropyl alcohol to effect extraction of any unbound DVBTP.

Three samples were prepared using standard techniques to evaluate the activity of the polymer bound DVBTP as an antioxidant synergist.

The testing procedure is described in detail in Industrial and Engineering Chemistry, Vol. 43, page 456

(1951) and Industrial and Engineering Chemistry, Vol. 45, page 392 (1953).

Sample A - SBR containing 0.5 phr polymer bound DVBTP required 13.4 hours to 1% oxygen absorption.

Sample B - SBR containing 0.5 phr Wingstay® C, (Trademark of The Goodyear Tire & Rubber Company for the reaction product of phenol, -methylstyrene and isobutylene, a commercially acceptable antioxidant) required 180.2 hours to 1% $O_2$ absorption.

Sample C - SBR containing 0.5 phr polymer bound DVBTP plus 0.5 phr Wingstay® C required 304.6 hours to 1% $O_2$ absorption.

This test amply demonstrates that polymer bound DVBTP exhibits synergism with an antioxidant in the stabilization of a polymer. Synergism is evidenced in that the stabilizing activity of Sample C is more than the additive effect seen in Sample A plus Sample B.

While certain representative embodiments and details have been shown for the purpose of illustrating the invention, it will be apparent to those skilled in this art that various changes and modifications may be made therein without departing from the scope of the invention.

CLAIMS

1. A process for the modification of a monomer having a vinyl moiety and a halo-benzyl moiety characterized by contacting said monomer with a quaternary ammonium or phosphonium salt and an alkaline ionizable molecule containing anti-degradant functionality and an alkaline compound at a temperature of 10° to 120°C and in the presence of an inert atmosphere.

2. The process of claim 1 characterized in that said quaternary ammonium or phosphonium salt is selected from the group of salts having the structural formula:

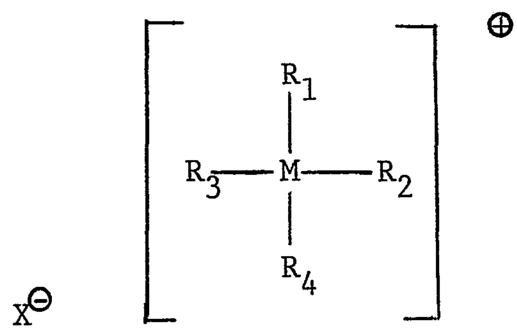

(I)

wherein M is nitrogen or phosphorus; $X^{\ominus}$ is selected from the group of radials consisting of chloride, bromide, fluoride, iodide, acetate, alkoxide and hydroxide; and $R_1$, $R_2$, $R_3$ and $R_4$ are mono-valent hydrocarbon radicals where the total number of carbon atoms in $R_1$, $R_2$, $R_3$ and $R_4$ is 12 to 70.

3. The process of claim 1 characterized in that reaction temperatures is from 60° to 80°C.

4. The process of claim 1 characterized in that said alkaline compound is a nonaqueous compound

selected from the group consisting of NaH, KH, LiH, Na, Li, K, NaOH, $NaHCO_3$, KOH, $K_2CO_3$, $Na_2CO_3$ and $K_2HCO_3$.

5. A process for the production of a functionalized monomer, wherein said monomer contains a polymerizable vinyl moiety and a halo-benzyl moiety, said process characterized by:

(A) contacting said monomer with a quaternary salt selected from the group of salts having the structural formula:

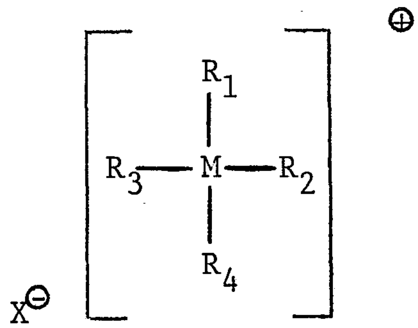

wherein M is nitrogen or phosphorous; $X^{\ominus}$ is selected from the group of radicals consisting of chloride, bromide, fluoride, iodide, acetate, alkoxide and hydroxide; $R_1$, $R_2$, $R_3$ and $R_4$ are mono- valent hydrocarbon radicals where the total number of carbon atoms in $R_1$, $R_2$, $R_3$ and $R_4$ is 12 to 70; and

(B) an alkali ionizable molecule selected from the group consisting of 4-mercaptopropionamide diphenylamine, 2,6-di-t-butyl-4-mercaptophenol, 4-mercaptophenol, p-hydroxydiphenylamine, N-(4-anilinophenyl)- 2-mercaptoacetamide, 2,6-di-t-butylhydroquinone, dodecyl mercaptopropionate; and, (C) an alkaline compound selected from the group consisting of NaOH, $NaHCO_3$, KOH, $K_2CO_3$, $Na_2CO_3$, $KHCO_3$, NaH, KH, LiH, Na, Li, K, NaOH, $K_2CO_3$, $Na_2CO_3$ and $K_2HCO_3$;

at a temperature of 10°-120°C in the presence of an inert atmosphere.